# EUROPÄISCHE PATENTANMELDUNG

(11) **EP 3 026 039 A1**
(43) Veröffentlichungstag der Anmeldung: **01.06.2016**
(21) Anmeldenummer: 14194734.1
(22) Anmeldetag: 25.11.2014
(51) Int. Cl.: C07C 67/20, C07C 69/675, B01J 38/04, B01J 38/06, B01J 38/12, B01J 38/16

(54) **Regenerierung von heterogenen Katalysatoren bei der Herstellung von alpha- oder beta-sustituierten Carbonsäureestern**

(71) Anmelder: Evonik Röhm GmbH, 64293 Darmstadt (DE)
(72) Erfinder: Krill, Dr. Steffen, 64367 Mühltal (DE); Treskow, Marcel, 64293 Darmstadt (DE); May, Dr. Alexander, 64342 Seeheim-Jugenheim (DE)

(57) **Zusammenfassung**

Die vorliegende Erfindung betrifft ein Verfahren zur Herstellung von alpha- oder beta-substituierten Carbonsäureestern durch Alkoholyse von alpha- oder beta-substituierten Carbonsäureamiden unter heterogener Katalyse, wobei der Katalysator durch ein Medium bei 250-600 °C regeneriert wird.

## Beschreibung

Die vorliegende Erfindung beschreibt ein Verfahren zur Regenerierung von heterogenen Katalysatoren bei der Herstellung von alpha- oder beta-sustituierten Carbonsäureestern (CSE) durch Umsetzen von Alkoholen mit alpha-, beta-substituierten Carbonsäureamiden (CA).

Die Alkoholyse von CA mittels heterogener Katalysatoren ist sowohl in der Flüssig- als auch in der Gas-Phase aus dem Stand der Technik hinlänglich bekannt. So werden z.B. in Bulletin of the Korean Chemical Society, 1997.18(11): p. 1208-1210 heterogenkatalytische Verfahren unter Einsatz von stark sauren Ionenaustauschern und Aluminiumoxid beschrieben.

In der EP 945423 werden neben der dominierenden homogenen Betriebsweise auch Beispiele mit unlöslichen Metalloxiden wie Wismuth- oder Ceriumoxid sowie metallischem Wismuth erwähnt.

JP 08073406 und JP 06345692 zielen ausschließlich auf eine heterogene Katalyse mit Metalloxiden, die sowohl ungeträgert als auch auf SiO₂-Trägern aufgebracht, eingesetzt werden. Als besonders geeignet werden hier Antimon-, Tellur-, Wismuth- oder Zirkoniumoxid genannt.

Die DE 102013213699, angemeldet am DPMA am12.07.2013, beschreibt ein entsprechendes Verfahren in der Flüssigphase, wobei bevorzugt in Reihe geschaltete Reaktoren mit insbesondere Y-, La- oder Si-dotierten Zirkoniumdioxiden betrieben werden.

Hauptproblem der genannten, im Wesentlichen kontinuierlich betriebenen Verfahren sind in der Regel die Standzeiten der Katalysatoren, deren Aktivitätsabfall sich zwar zwischenzeitlich durch Erhöhung der Reaktionstemperatur beheben läßt, die aber letztendlich dann doch ausgetauscht bzw. aufwendig, in der Regel extern regeneriert werden müssen. Bei den meist teuren heterogenen Katalysatoren bedeutet dies einen nicht unwesentlichen technischen und wirtschaftlichen Aufwand.

Aufgabe der vorliegenden Erfindung war es daher, ein Verfahren zur Herstellung von CSE durch Alkoholyse eines CA zur Verfügung zu stellen, wobei der heterogene Katalysator möglichst schnell, kostengünstig, effizient und ohne längere Stillstandszeiten regeneriert werden kann. Überraschend wurde nun gefunden, dass nachdem weder alleiniger Abbrand noch Extraktion mit Lösemitteln zu einem gewünschten Regenerationsergebnis führten, die zuvor genannten, sowie weitere nicht explizit genannte Aufgaben gelöst werden durch die Bereitstellung eines Verfahrens zur Herstellung von CSE durch Alkoholyse des entsprechenden CA, dadurch gekennzeichnet, dass der heterogene Katalysator durch aktive oder passive Behandlung mit einem Medium bei 250-600 °C regeneriert wird.

Die Vorteile des erfindungsgemäßen Verfahrens bestehen vor allem darin, dass die Regenerierung ohne Ausbau des Katalysators erfolgen kann und keine Abbrand-Bedingungen vorliegen müssen.

Das erfindungsgemäße Verfahren betrifft Alkoholysen von CA, die sowohl in flüssiger als auch in gasförmiger Phase unter heterogener Katalyse durchgeführt werden.

Geeignete Katalysatoren für das erfindungsgemäße Verfahren sind Zirkoniumdioxid und Zirkoniumdioxide, die mit Elementen der 2.-4., der 7. sowie der 9.-13. Gruppe des Periodensystems bzw. mit La, Sb oder Bi dotiert sind. Bevorzugte Dotierungselemente für Zirkoniumdioxid stammen aus der 3., 7., 9., 10. oder 13. Gruppe des Periodensystems bzw. sind ausgewählt aus der Gruppe bestehend aus B, Al, Mn, Co, Ni, Y, La oder Yb. Besonders bevorzugt sind die Dotierungselemente Ce, K, La, Mo, P, S, Si, Ti, W, Y oder Zn, und ganz besonders bevorzugt Ce, K, Si, Y oder La. Der Dotierungsgehalt beträgt 0,1-50, bevorzugt 0,2-20, besonders bevorzugt 0,4-15 Gew%.

Zu den in der Reaktion der Erfindung einsetzbaren CA gehören üblicherweise all diejenigen Carbonsäureamide, die in alpha-oder beta Stellung zur Carbonsäureamidgruppe wenigstens eine Hydroxygruppe aufweisen

Als Edukte für dieses Verfahren eignen sich Carbonsäureamide der Formel (1): 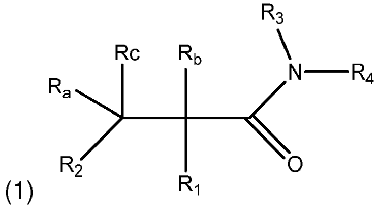 wobei R₁ = H oder CHR'R"; R₂= Kohlenstoffrest mit 1-7 Kohlenstoffatomen, linear, verzweigt oder alicylisch und Rₐ, R_{b} und R_{c} unabhängig voneinander jeweils einen Rest ausgewählt aus der Gruppe Wasserstoff, Kohlenstoffrest mit 1-7 Kohlenstoffatomen, linear, verzweigt oder alicyclisch oder einer Abgangsgruppe in alpha-Stellung zu Wasserstoff darstellen.

Carbonsäureamide sind in der Fachwelt allgemein bekannt. Üblicherweise werden hierunter Verbindungen mit Gruppen der Formel CONR³R⁴, worin R³ und R⁴ unabhängig Wasserstoff oder eine 1-30 Kohlenstoffatome aufweisende Gruppe darstellt, die insbesondere 1-20, bevorzugt 1-10 und insbesondere 1-5 Kohlenstoffatome umfasst, wobei Amide mit R³ und R⁴ gleich Wasserstoff besonders bevorzugt sind.

Das Carbonsäureamid kann 1, 2, 3, 4 oder mehr Gruppen der Formel -CONR³R⁴ umfassen. Hierzu gehören insbesondere Verbindungen der Formel R(-CONR³R⁴)n, worin der Rest R eine 1-30 Kohlenstoffatome aufweisende Gruppe darstellt, die insbesondere 1-20, bevorzugt 1-10, insbesondere 1-5 und besonders bevorzugt 2-3 Kohlenstoffatome umfasst, R³ und R⁴ die zuvor genannte Bedeutung hat und n eine ganze Zahl im Bereich von 1-10, vorzugsweise 1-4 und besonders bevorzugt 1 (Monomere-) oder 2 (Dimere-Amide) darstellt.

Der Ausdruck "1 bis 30 Kohlenstoffatome aufweisende Gruppe" kennzeichnet Reste organischer Verbindungen mit 1 bis 30 Kohlenstoffatomen. Er umfasst neben aromatischen und heteroaromatischen Gruppen auch aliphatische und heteroaliphatische Gruppen, wie beispielsweise Alkyl-, Cycloalkyl-, Alkoxy-, Cycloalkoxy-, Cycloalkylthio- und Alkenylgruppen. Dabei können die genannten Gruppen verzweigt oder nicht verzweigt sein.

Erfindungsgemäß bezeichnen aromatische Gruppen Reste ein oder mehrkerniger aromatischer Verbindungen mit vorzugsweise 6 bis 20, insbesondere 6 bis 12 C-Atomen.

Heteroaromatische Gruppen kennzeichnen Arylreste, worin mindestens eine CH-Gruppe durch N ersetzt ist und/oder mindestens zwei benachbarte CH-Gruppen durch S, NH oder O ersetzt sind.

Erfindungsgemäß bevorzugte aromatische oder heteroaromatische Gruppen leiten sich von Benzol, Naphthalin, Biphenyl, Diphenylether, Diphenylmethan, Diphenyldimethylmethan, Bisphenon, Diphenylsulfon, Thiophen, Furan, Pyrrol, Thiazol, Oxazol, Imidazol, Isothiazol, Isoxazol, Pyrazol, 1,3,4-Oxadiazol, 2,5- Diphenyl-1,3,4-oxadiazol, 1,3,4-Thiadiazol, 1,3,4-Triazol, 2,5-Diphenyl-1,3,4-triazol, 1,2,5-Triphenyl-1,3,4-triazol, 1,2,4-Oxadiazol, 1,2,4-Thiadiazol, 1,2,4-Triazol, 1,2,3- Triazol, 1,2,3,4-Tetrazol, Benzo[b]thiophen, Benzo[b]furan, Indol, Benzo[c]thiophen, Benzo[c]furan, Isoindol, Benzoxazol, Benzothiazol, Benzimidazol, Benzisoxazol, Benzisothiazol, Benzopyrazol, Benzothiadiazol, Benzotriazol, Dibenzofuran, Dibenzothiophen, Carbazol, Pyridin, Bipyridin, Pyrazin, Pyrazol, Pyrimidin, Pyridazin, 1,3,5-Triazin, 1,2,4-Triazin, 1,2,4,5-Triazin, Tetrazin, Chinolin, Isochinolin, Chinoxalin, Chinazolin, Cinnolin, 1,8-Naphthyridin, 1,5-Naphthyridin, 1,6-Naphthyridin, 1,7- Naphthyridin, Phthalazin, Pyridopyrimidin, Purin, Pteridin oder Chinolizin, 4H-Chinolizin, Diphenylether, Anthracen, Benzopyrrol, Benzooxathiadiazol, Benzooxadiazol, Benzopyridin, Benzopyrazin, Benzopyrazidin, Benzopyrimidin, Benzotriazin, Indolizin, Pyridopyridin, Imidazopyrimidin, Pyrazinopyrimidin, Carbazol, Aciridin, Phenazin, Benzochinolin, Phenoxazin, Phenothiazin, Acridizin, Benzopteridin, Phenanthrolin und Phenanthren ab, die gegebenenfalls auch substituiert sein können.

Zu den bevorzugten Alkylgruppen gehören die Methyl-, Ethyl-, Propyl-, Isopropyl-, 1- Butyl-, 2-Butyl-, 2-Methylpropyl-, tert.-Butyl-, Pentyl-, 2-Methylbutyl-, 1,1- Dimethylpropyl-, Hexyl-, Heptyl-, Octyl-, 1,1,3,3-Tetramethylbutyl, Nonyl-, 1-Decyl-, 2- Decyl-, Undecyl-, Dodecyl-, Pentadecyl- und die Eicosyl-Gruppe.

Zu den bevorzugten Cycloalkylgruppen gehören die Cyclopropyl-, Cyclobutyl-, Cyclopentyl-, Cyclohexyl-, Cycloheptyl- und die Cyclooctyl-Gruppe, die gegebenenfalls mit verzweigten oder nicht verzweigten Alkylgruppen substituiert sind.

Zu den bevorzugten Alkenylgruppen gehören die Vinyl-, Allyl-, 2-Methyl-2-propen-, 2- Butenyl-, 2-Pentenyl-, 2-Decenyl- und die 2-Eicosenyl-Gruppe.

Zu den bevorzugten heteroaliphatischen Gruppen gehören die vorstehend genannten bevorzugten Alkyl- und Cycloalkylreste, in denen mindestens eine Kohlenstoff-Einheit durch O, S oder eine Gruppe NR8 oder NR8R9 ersetzt ist und R8 und R9 unabhängig eine 1 bis 6 Kohlenstoffatome aufweisende Alkyl-, eine 1 bis 6 Kohlenstoffatome aufweisende Alkoxy- oder eine Arylgruppe bedeutet.

Erfindungsgemäß ganz besonders bevorzugt weisen die Carbonsäureamide verzweigte oder nicht verzweigte Alkyl-, oder Alkoxygruppen mit 1 bis 20 Kohlenstoffatomen, vorzugsweise 1 bis 12, zweckmäßigerweise 1 bis 6, insbesondere 1 bis 4 Kohlenstoffatomen und Cycloalkyl- bzw. Cycloalkyloxygruppen mit 3 bis 20 Kohlenstoffatomen, vorzugsweise 5 bis 6 Kohlenstoffatomen auf.

Der Rest R kann Substituenten aufweisen. Zu den bevorzugten Substituenten gehören u.a. Halogene, insbesondere Fluor, Chlor, Brom, sowie Pseudohalogenide (beispielsweise CN⁻, SCN⁻, NCS⁻), Alkoxy-, Carboxy oder Hydroxyreste.

Die HCA können beim Verfahren der Erfindung einzeln oder als Mischung von zwei oder drei oder mehreren unterschiedlichen HCA eingesetzt werden. Zu besonders bevorzugten HCA gehören alpha- oder beta-Hydroxyisobuttersäureamid und/oder alpha- oder beta-Hydroxyisopropionsäureamid.

Weiterhin ist es in einer Abwandlung des erfindungsgemäßen Verfahrens von besonderem Interesse solche HCA einzusetzen, die durch Cyanhydrinsynthese aus Ketonen oder Aldehyden und Blausäure zugänglich sind. In einem ersten Schritt wird hierbei die Carbonylverbindung, beispielsweise ein Keton, insbesondere Aceton, oder ein Aldehyd, beispielsweise Acetaldehyd, Propanal, Butanal, mit Blausäure zum jeweiligen Cyanhydrin umgesetzt. Besonders bevorzugt wird hierbei Aceton und/oder Acetaldehyd auf typische Weise unter Verwendung einer geringen Menge an Alkali oder eines Amins als Katalysator umgesetzt. In einem weiteren Schritt wird das so erhaltene Cyanhydrin mit Wasser zum HCA umgesetzt.

Zu den bei Verfahren der Erfindung mit Erfolg einsetzbaren Alkoholen gehören alle dem Fachmann geläufigen Alkohole sowie Vorläuferverbindungen von Alkoholen, die unter den angegebenen Bedingungen von Druck und Temperatur in der Lage sind, mit den HCA im Sinne einer Alkoholyse zu reagieren. Bevorzugt erfolgt die Umsetzung des HCA durch Alkoholyse mit einem Alkohol, der vorzugsweise 1-10 Kohlenstoffatome, besonders bevorzugt 1 bis 5 Kohlenstoffatome umfasst. Bevorzugte Alkohole sind u.a. Methanol, Ethanol, Propanol, Butanol, insbesondere n-Butanol und 2-Methyl-1-propanol, Pentanol, Hexanol, Heptanol, 2-Ethylhexanol, Octanol, Nonanol und Decanol. Besonders bevorzugt wird als Alkohol Methanol und / oder Ethanol eingesetzt, wobei Methanol ganz besonders zweckmäßig ist. Auch der Einsatz von Vorstufen eines Alkohols ist prinzipiell möglich. So können beispielsweise Alkylformiate eingesetzt werden. Insbesondere eignen sich Methylformiat oder eine Mischung von Methanol und Kohlenmonoxid.

Des Weiteren sind Verfahren bevorzugt, die dadurch gekennzeichnet sind, dass man als HCA alpha Hydroxyisobuttersäureamid (HIBA) und als Alkohol Methanol einsetzt, die dann zum Zielprodukt alpha -Hydroxyisobuttersäuremethylester (HIBSM) reagieren.

Das erfindungsgemäße Verfahren läßt sich in allen aus dem Stand der Technik bekannten Reaktortypen durchführen. Bevorzugt sind Rohrreaktoren, Rohrbündelreaktoren und Wirbelschichtreaktoren.

Das erfindungsgemäße Verfahren läßt sich durch Aufheizen des Reaktors auf Regenerationsbedingung durchführen. Dies kann durch elektrische Heizungen, Salzbad oder Dampf erfolgen.

Bevorzugt lässt sich das erfindungsgemäße Verfahren durchführen, indem der Reaktor durch einen Gasstrom beheizt wird. Geeignete Regenerationsmedien (Gase) sind Luft, Wasserdampf, inerte Gase, wie z.B. Helium, Stickstoff, Argon, Xenon (Edelgase), oder Verbrennungsabgase wie z.B.CO, CO₂ oder Stickoxide, oder Erdgas, oder Wasserstoff, oder niedrig siedende Kohlenwasserstoffe der allgemeinen Formeln CₙH₂ₙ₊₂ (Alkane), CₙH₂ₙ (Alkene), CₙH₂ₙ₋₂ (Alkine), und n eine ganze Zahl im Bereich von 1-10, vorzugsweise 1-4 und besonders bevorzugt 1 oder 2 darstellt, oder einwertige Alkanole der Formel CₘH₂ₘ₊₁OH, oder einwertige Alkanale der Formel CₘH₂ₘ₊₁CHO, oder einwertige Alkansäuren der Formel CₘH₂ₘ₊₁COOH bei denen m eine ganze Zahl im Bereich von 0-10, vorzugsweise 0-4 und besonders bevorzugt 1 oder 2 darstellt, sowie Mischungen derselben. Die belasteten bzw. verbrauchten Regenerationsmedien können geeigneten üblichen Entsorgungsverfahren wie z.B. einer biologischen Kläranlage oder einem Thermal Oxidizer zugeführt werden.

Das erfindungsgemäße Verfahren wird ganz besonders bevorzugt mit einem Medium durchgeführt, das einen Siedepunkt unterhalb von 150 °C hat, insbesondere Luft, Wasserdampf oder Stickstoff oder deren Mischungen.

Das erfindungsgemäße Verfahren kann sowohl unter reduktiven als auch unter oxidativen Bedingungen durchgeführt werden. Im erfindungsgemäßen Verfahren jedoch bevorzugt sind reduktive Bedingungen, da unter oxidativen Bedingungen, besonders wenn die Regeneration "in situ", d.h. innerhalb der Verfahrensaggregate erfolgen soll, eine "run-away"-Reaktion auftreten kann, wobei der üblicherweise zu beobachtende Temperaturanstieg nicht mehr zu beherrschen ist.

Die Regenerationstemperaturen betragen 250-600, bevorzugt 300-500, besonders bevorzugt 350-450 °C.

Die Dauer der Regeneration beträgt 0,1-168, bevorzugt 4-72, besonders bevorzugt 4-36 h.

Erfindungsgemäß kann der heterogene Katalysator durch aktive oder passive Behandlung mit einem Medium bei 250-600 °C regeneriert wird. Passiv bedeutet, das im System vorhandene Medium, oder Mischungen von Medien werden zur Regenerierung genutzt. Aktiv bedeutet, dass ein geeignetes Medium oder eine Mischung aus verschiedenen Medien zur Regeneration des Katalysators zugeführt wird.

Die WHSV (weight hourly space velocity) des Regenerationsmediums am zu regenerierenden Katalysator beträgt 0-5, bevorzugt 0,01-5, besonders bevorzugt 0,05-1,5, ganz besonders bevorzugt 0,1-0,9 h⁻¹.

Die folgenden Beispiele sollen die Erfindung erläutern aber in keiner Weise beschränken.

### Beispiel 1: Herstellung von 2-HIBSM

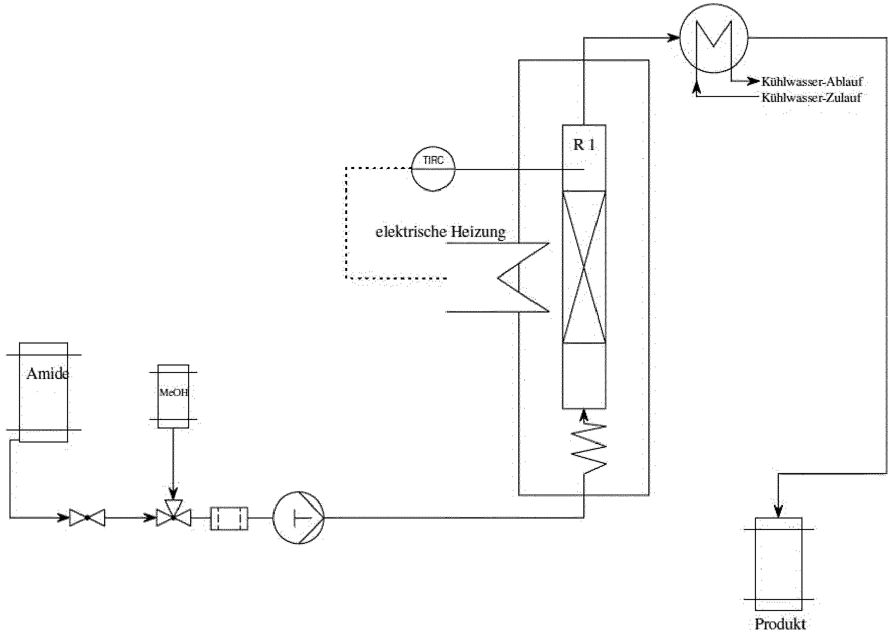

Es wird eine 30 Gew.-%ige 2-HIBA-Lösung in Methanol mit 16 g eines Yttrium-dotierten Zirkonoxid Katalysators umgesetzt. Die Methanolyse wird bei einem Druck von ca. 70 bar, einem Volumenstrom von ca. 2 ml min⁻¹ und einer modifizierten Verweilzeit von 8 g min ml⁻¹ durchgeführt. Das aus dem Rohrreaktor austretende Produktgemisch wird durch den mit Wasser gekühlten Rohrschlangen-Wärmetauscher auf Raumtemperatur abgekühlt und gelangt in den Auffangbehälter. Die Entnahme einer Produkt- oder Eduktprobe ist vor und nach dem Reaktor durch entsprechende Ventile möglich. Die Betriebstemperatur in der Methanolyseanlage beträgt 200 °C.

### Vergleichsbeispiele 1-4: Regeneration durch Extraktion

Ein aus einer Anlage analog Fig. 1 nach ca. 9000 h TOS (time on stream) ausgeschleuster, Yttriumdotierter Zirkoniumdioxid Katalysator mit einer stationären Ausgangsaktivität von 19 % Ausbeute bezogen auf HIBSM und einer verbliebenen Restaktivität von 10,9 % Ausbeute bezogen auf HIBSM wurde in einer Soxhlet-Aufsatz mit unterschiedlichen Extraktionsmitteln behandelt und in den Reaktor zurückgeführt. Die resultierenden Ausbeuten bezogen auf HIBSM (100h nach der Regeneration) der so regenerierten Katalysatoransätze sind in Tab. 1 dargestellt. Die Ursprungsaktivität des Katalysators kann nur ungenügend wieder hergestellt werden.

**Tab. 1: Ausbeuten nach Extraktion (als Maximalwerte gezeigt)**

| Vergleichsbeispiel | | Ausbeute HIBSM |
|---|---|---|
| | Frischkat (stationäre Phase) | 19 |
| | vor Extraktion | 10,9 |
| 1 | Wasser | 12,3 |
| 2 | Methanol | 10,8 |
| 3 | Toluol | 9,9 |
| 4 | Hexan | 8,5 |

### Vergleichsbeispiel 5: Regeneration durch Abbrand

Ein aus einer Anlage analog Fig. 1 nach ca. 9000 h TOS (time on stream) ausgeschleuster, Yttriumdotierter Zirkoniumdioxid Katalysator mit einer Ausgangsaktivität von 19 % Ausbeute bezogen auf HIBSM und einer verbliebenen Restaktivität von 10,9 % Ausbeute bezogen auf HIBSM wurde in einem Ofen unter Sauerstoffzufuhr abgebrannt bis kein Sauerstoff mehr verbraucht wurde und anschließend in den Reaktor zurückgeführt. Die resultierenden Ausbeuten bezogen auf HIBSM der so regenerierten Katalysatoransätze sind in Tab. 2 dargestellt. Die Ursprungsaktivität des Katalysators kann nur ungenügend wieder hergestellt werden.

**Tab. 2: Ausbeuten nach Abbrand**

| TOS [h] | Umsatz [%] |
|---|---|
| 24 | 12,2 |
| 47 | 12,0 |
| 89,5 | 9,5 |
| 114 | 10,4 |
| 138 | 11,0 |
| 161,25 | 11,1 |
| 186 | 11,6 |

### Beispiel 2-5: Unterschiedliche Regenerationsmedien

Der gleiche wie in den Vergleichsbeispielen 1-5 nach 9000 h entnommene verbrauchte Katalysator wurde mit verschiedenen Regenerationsmedien jeweils 4 h bei 400 °C behandelt und danach wieder in den Reaktor eingespeist. Die Ausbeuten bzgl. HIBSM sind in Tab. 3 für verschiedene Betriebszeiten dargestellt. Die ursprüngliche Aktivität des Frischkatalysators konnte wieder eingestellt werden.
Der Katalysator hatte vor Regeneration jeweils eine Aktivität von 10,9 %.

**Tab. 3: Abhängigkeit der Ausbeute von der Betriebszeit bei unterschiedlichen Regenerationsmedien**

| | Beispiel | 2 | 3 | 4 | 5 |
|---|---|---|---|---|---|
| TOS [h] | Frischkatalysator | Luft | Stickstoff | Wasserdampf | Luft + Wasser |
| 3 | | | 24,3% | | 24,8% |
| 5 | 29,7% | | | | |
| 6 | | 23,5% | | | |
| 21 | 27,2% | | | | |
| 22 | | | 22,2% | 23,6% | |
| 27 | | | | | 21,9% |
| 30 | | 21,9% | | | |
| 46 | 24,6% | | | 23,0% | |
| 47 | | | 21,4% | | |
| 51 | | | | | 21,0% |
| 54 | | 21,6% | | | |
| 69 | 22,6% | | | | |
| 70 | | | 20,6% | | |
| 71 | | | | 22,5% | |
| 94 | 21,4% | | 20,1% | | |
| 118 | | 20,5% | | | 20,1% |
| 141 | | 21,0% | | | |
| 143 | | | | | 19,9% |
| 151 | | | | 21,4% | |
| 163 | 19,0% | | | | |
| 166 | | | 19,3% | | |
| 167 | | | | 21,2% | |
| 187 | 19,4% | | | | |
| 190 | | | | 21,1% | |
| 207 | 20,5% | | | | |
| 212 | | | | 21,9% | |
| 239 | 19,8% | | | | |
| 262 | 19,7% | | | | |
| 284 | 19,5% | | | | |
| 303 | | | | 21,3% | |
| 304 | 18,6% | | | | |
| 328 | | | | 21,3% | |
| 331 | 18,8% | | | | |
| 351 | 19,1% | | | 20,9% | |

### Beispiele 6-8, Vergleichsbeispiele 6-7: Unterschiedliche Regenerationstemperaturen

Analog zu Beispiel 4 wurde der ausgeschleuste Katalysator mit Dampf bei unterschiedlichen Regenerationstemperaturen für 4h behandelt. Die Ergebnisse bzgl. Ausbeute an HIBSM in Abhängigkeit von der Betriebsdauer sind in Tab. 4 aufgelistet. Wie unschwer zu erkennen, beginnt die Regeneration bei 250 °C, jedoch werden sinnvolle Regenerationseffekte erst ab 300 °C erreicht.

**Tab. 4: Abhängigkeit der Ausbeute von der Betriebszeit bei unterschiedlichen Regenerationstemperaturen**

| | | Beispiel 6 | Beispiel 7 | Beispiel 8 | Vergleichsbeispiel 6 | Vergleichsbeispiel 7 |
|---|---|---|---|---|---|---|
| TOS / h | Frischkatalysator | 400 °C | 350 °C | 300 °C | 250 °C | 200 °C |
| 1 | | 25,9% | | | | |
| 3 | | | | 18,8% | 12,5% | 12,2% |
| 4 | | 26,5% | | | | |
| 5 | 29,7% | | | | | |
| 20 | | | 22,0% | | | |
| 21 | 27,2% | | | | | |
| 28 | | 23,7% | | | | |
| 29 | | | | | | 14,4% |
| 42 | | | | | 13,5% | |
| 45 | | | | 17,5% | | |
| 46 | 24,6% | | | | | |
| 52 | | 22,8% | | | | |
| 53 | | | | | | 13,9% |
| 69 | 22,6% | | | 17,1% | | |
| 71 | | | | | | 13,0% |
| 90 | | | | | 13,3% | |
| 93 | | | | 17,3% | | |
| 94 | 21,4% | | | | | |
| 100 | | | 21,3% | | | |
| 113 | | | | | 13,1% | |
| 116 | | | 21,3% | | | |
| 117 | | | | 16,8% | | |
| 120 | | 21,2% | | | | |
| 139 | | | 21,0% | | | |
| 142 | | 20,6% | | | | |
| 163 | 19,0% | | | | | |

### Beispiele 9-14: Unterschiedliche Wasserdampfmengen

Analog zu Beispiel 4 wurde der zu regenerierende Katalysator mit unterschiedlichen Wasserdampfmengen bei 350 °C jeweils 4 h beaufschlagt und die Ausbeutewerte bzgl. HIBSM in der beschriebenen Anlage ermittelt. Die Werte sind in Tab. 5 zusammen gestellt. Für WHSV-Werte (weight hourly space velocity) von 0,3-1,0 pro h werden keine wesentlichen Unterschiede festgestellt.

**Tab. 5: Regeneration mit unterschiedlichen Wasserdampfmengen**

| | Beispiel | 9 | 10 | 11 | 12 | 13 | 14 |
|---|---|---|---|---|---|---|---|
| | | WHSV / h⁻¹ | | | | | |
| TOS / h | Frischkatalysator | 0 | 0,3 | 0,4 | 0,5 | 0,6 | 1 |
| 3 | | 22,5% | 21,3% | | | | 22,5% |
| 5 | 29,7% | | | | | | |
| 19 | | 21,4% | | 21,5% | 21,4% | 23,1% | 21,4% |
| 20 | | | 19,2% | | | | |
| 21 | 27,2% | | | | | | |
| 43 | | | | 19,8% | 20,1% | 22,1% | |
| 44 | | | 19,0% | | | | |
| 46 | 24,6% | | | | | | |
| 67 | | | | 19,0% | 19,3% | 21,1% | |
| 69 | 22,6% | | | | | | |
| 91 | | 19,6% | | | | | 19,6% |
| 94 | 21,4% | | | | | | |
| 115 | | 19,5% | | | | | 19,5% |
| 116 | | | 17,8% | | | | |
| 138 | | | | 17,5% | 18,1% | 20,2% | |
| 139 | | 19,4% | | | | | 19,4% |
| 140 | | | 17,4% | | | | |
| 162 | | | | 17,2% | 17,9% | 19,6% | |
| 163 | 19,0% | 18,9% | | | | | 18,9% |
| 164 | | | 17,5% | | | | |
| 187 | 19,4% | | | 16,9% | 17,7% | 19,9% | |
| 207 | 20,5% | | | | | | |
| 211 | | | | 16,6% | 17,3% | | |
| 239 | 19,8% | | | | | | |

## Patentansprüche

1. Verfahren zur Herstellung von alpha- oder beta-substituierten Carbonsäureestern durch Alkoholyse des entsprechenden alpha- oder beta- substituierten Carbonsäureamids mittels heterogener Katalyse **dadurch gekennzeichnet, dass** der heterogene Katalysator durch aktive oder passive Behandlung mit einem Medium oder einer Mischung von Medien bei 250-600 °C regeneriert wird.

2. Verfahren nach Anspruch 1 **dadurch gekennzeichnet, dass** aktiv mit einem Medium und einer WHSV (weight hourly space velocity) von 0,01-5 h⁻¹ regeneriert wird.

3. Verfahren nach Anspruch 1 **dadurch gekennzeichnet, dass** das Medium Luft, Stickstoff, Wasserdampf oder deren Mischungen enthält.

4. Verfahren nach einem der vorhergehenden Ansprüche **dadurch gekennzeichnet, dass** die Regenerationszeit 0,1-168 h beträgt.

5. Verfahren nach einem der vorhergehenden Ansprüche **dadurch gekennzeichnet, dass** die Regenerationszeit 4-36 h beträgt.

6. Verfahren nach einem der vorhergehenden Ansprüche **dadurch gekennzeichnet, dass** die WHSV des Regenerationsmediums am zu regenerierenden Katalysator 0,1-0,9 h⁻¹ beträgt.

7. Verfahren nach einem der vorhergehenden Ansprüche **dadurch gekennzeichnet, dass** als Katalysator ein Y-, La- oder Si-dotiertes Zirkoniumdioxid eingesetzt wird.
